# EUROPEAN PATENT SPECIFICATION

(11) **EP 1 140 974 B1**
(45) Date of publication and mention of the grant of the patent: **02.03.2005**
(21) Application number: 99967436.9
(22) Date of filing: 20.12.1999
(51) Int. Cl.: C07K 1/00, A61K 39/12, C12N 7/00, C12N 1/20, C12N 15/09, C12Q 1/70, G01N 33/53

(54) **NEUTRALIZING ASSAY USING HUMAN PAPILLOMAVIRUS VIRUS-LIKE PARTICLES**
NEUTRALSIERUNGS-ASSAY, DAS HUMANE PAPILLOMAVIRUS-ÄHNLICHE PARTIKEL VERWENDET
DOSAGE NEUTRALISANT UTILISANT DES PARTICULES VERIFORMES DU PAPILLOMAVIRUS HUMAIN

(30) Priority: 23.12.1998 US 113388 P
(43) Date of publication of application: 10.10.2001
(73) Proprietor: Merck & Co., Inc., Rahway, New Jersey 07065-0907 (US)
(72) Inventor: JANSEN, Kathrin, U., Rahway, NJ 07065-0907 (US); YEAGER, Mark, D., Rahway, NJ 07065-0907 (US); KELLER, Paul, M., Rahway, NJ 07065-0907 (US); LOWE, Robert, S., Rahway, NJ 07065-0907 (US); ASTE-AMEZAGA, Miguel, Rahway, NJ 07065-0907 (US)
(74) Representative: Horgan, James Michael Frederic
(86) International application number: PCT/US1999/030297
(87) International publication number: WO 2000/039151

(56) References cited:
- US-A- 5 795 754
- US-A- 5 952 216
- US-A- 6 013 262
- RODEN R B S ET AL: "IN VITRO GENERATION AND TYPE-SPECIFIC NEUTRALIZATION OF A HUMAN PAPILLOMAVIRUS TYPE 16 VIRION PSEUDOTYPE" JOURNAL OF VIROLOGY, THE AMERICAN SOCIETY FOR MICROBIOLOGY, US, vol. 70, no. 9, 1 September 1996 (1996-09-01), pages 5875-5883, XP002048982 ISSN: 0022-538X
- KAWANA K ET AL: "In vitro construction of Pseudovirions of human papillomavirus type 16: incorporation of plasmid DNA into reassembled L1/L2 capsids" JOURNAL OF VIROLOGY, THE AMERICAN SOCIETY FOR MICROBIOLOGY, US, vol. 72, no. 12, December 1998 (1998-12), pages 10298-10300, XP002231121 ISSN: 0022-538X

## Description

### FIELD OF THE INVENTION

This invention is related to a novel method of detecting human papillomavirus (HPV) neutralizing antibodies in a serum or plasma.

### BACKGROUND OF THE INVENTION

Human Papillomaviruses (HPV) infect the genital tract and has been associated with various dysplasias, cancers and other diseases. These diseases are currently targets for vaccine developments, and vaccines based on virus-like particles (VLPs) which contain only L1 or L1+L2 capsid proteins are currently in trials.

To test vaccine candidates, neutralization assays would be desirable. While there are over 100 HPV types described today, there are only a few HPV types for which neutralization assays exist. Since HPV does not grow in tissue culture, the development of neutralization assays for HPV has been difficult.

To facilitate vaccine development, it would be desirable to develop a basic neutralization assay which could be easily modified so as to have a neutralization assay for any HPV type.

Roden *et al*., J. Virol., 1996, 5875-5883 and Kawana *et al*., J. Virol., 1998, 10298-10300 disclose an assay to determine whether anti-HPV neutralising antibodies are present in a sample.

US-A-5795754 (Merck & Co., Inc.) discloses synthetic VLPs and assays using them to validate VLPs manufactured through recombinant DNA technologies.

### DESCRIPTION OF THE INVENTION

This invention relates to a papillomavirus "pseudovirion" comprising a virus-like particle (VLP) which is covalently linked to a reporter gene DNA construct. In preferred embodiments, the VLPs are human papillomavirus (HPV) VLPs.

This invention also includes assays to determine if an anti-VLP antibody is present in a sample comprising the steps of: combining a cell culture susceptible to infection by the pseudovirions, the sample, and pseudovirions; and measuring the amount of uptake of the pseudovirions by the cell. The uptake may be compared to a control where no sample is present, or the sample is a pre-immune sample, wherein a decrease in cell uptake of pseudovirions indicates the presence of neutralizing antibodies in the sample.

Another aspect of this invention is a method of making the pseudovirion construct, comprising generating relatively pure VLPs and attaching the reporter gene construct with a heterobifunctional cross-linker.

Yet another aspect of this invention is a method of delivery of a reporter genes into a cell comprising infecting a cell with a pseudovirion.

### DESCRIPTION OF THE DRAWINGS:

Figure 1 is a diagram showing a reporter gene construct based on the beta-lactamase gene (BLAM).
Figure 2 shows the inhibition of HPV16 pseudovirion uptake into cells by HPV16 neutralizing monoclonal antibody H16.V5.
Figure 3 shows the inhibition of HPV 16 pseudovirion uptake into cells by neutralizing antibody in the sera of individuals immunized with HPV16 VLPs.

As used throughout the specification and claims, the following definitions apply:
"Pseudovirion" means a papillomavirus virus-like particle which is chemically linked or bonded to a reporter gene construct, either directly or through a linking group.
"Reporter gene construct" means any nucleic acid encoding a protein whose transcription, translation, or post-translation activity can be conveniently detected, along with known necessary elements required for control of its transcription and translation (including, for example, promoters, enhancers, transcription termination sequences, and the like). Examples of reporter gene constructs include genes for beta-galactosidase, luciferase, and green-fluorescent protein, and the like.

### DETAILED DESCRIPTION OF THE INVENTION

The pseudovirions and methods of using them do not depend on any particular papillomavirus strain or any other viral capsid. In preferred embodiments, the papillomavirus is a human papillomavirus (HPV), and it is particularly preferred that the HPV is one of the strains which is associated with genital warts and/or genital cancers, and may be selected from the group consisting of: HPV6a, HPV6b, HPV11, HPV16, HPV18, HPV31, HPV33, HPV35, HPV42, HPV43, HPV44, HPV45, HPV51, HPV52, and HPV56.

A pseudovirion can be made from virtually any papillomavirus VLP. A wild-type VLP generally is made up predominantly of L1 and a minor amount of L2 protein. However, it is known that VLPs may contain only L1 protein. In addition, VLPs have been described which contain modified L1 protein, modified L2 proteins, proteins (whether or not naturally occurring, such as HPV E-proteins) which have been fused to at least a portion of the L1 or L2 proteins, and/or an additional, non-naturally occurring protein. For purposes of this invention, any of the foregoing may be used as a starting material for the construction of pseudovirions. Generally, preferred VLPs contain either L1 or the combination of L1 and L2 ("L1+L2") protein.

The L1 or L1+L2 protein-containing VLPs can be made by transforming a selected host cell with genes encoding the L1 or L1+L2 proteins. General techniques, the genes for various types of L1 and L2 , and methods for recombinant expression of L1 and L2 proteins are known in the art, and may be used. Preferred host cells include yeast, insect cells, mammalian cells and *E. coli*.

Under one preferred embodiment of this invention, yeast are transformed with plasmid DNA containing the genes encoding L1 protein under control of known yeast promoters, such as the yeast Gal 1 and Gal 10 promoters. Expression of the gene product is induced by addition of galactose to the growth media, and the VLPs are isolated from the induced cell lysates.

Virtually any reporter gene construct can be coupled to the VLPs to make a pseudovirion. One preferred reporter gene construct used is the beta-lactamase gene ("BLAM"), but other reporter constructs encoding enzymes or proteins like beta-galactosidase, luciferase and green-fluorescent protein or genes coding for spliced transcripts can also be used.

The reporter construct may be coupled to the VLP using known coupling reactions. In a preferred embodiment, the reporter gene construct is attached to a linker which is commercially available, and the linker is attached to the VLP. A preferred cross-linking reagent is (succinimidyl-maleimidomethyl-cyclohexane-carboxylate "SMCC"). Other potentially useful crosslinking reagants in addition to SMCC would have the following properties: 1) heterobifunctional or sequentially activated crosslinkers that would eliminate interfering oligo-oligo or VLP-VLP crosslinking, 2) stable reactions with either 5'-amino or 5'-thiol modified oligonucleotides, 3) stable reactions with primary amine, free sulfhydryl, or carbohydrate moieties on the VLP protein. These reagents would include, but are not limited to the following and their sulfated derivatives: SMPB (succinimidyl-4-(p-maleimidophenyl) butyrate), SIAB (succinimidyl(4-iodoacetyl) amino benzoate), GMBS (n-(4-maleimido-butyryloxy)-succinimide), MBS (m-maleimidobenzoyl sulfosuccinimide ester), and MPBH (4-(4-N-maleimidophenyl) butyric acid hydrazide hydrochloride).

In constructing such a linker, molar ratio of reporter gene constructs to the VLP may vary. In accordance with this invention, the ratio of reporter gene construct to VLP should be from about 0.1 to 50, preferably from about 1 to 5, and more preferably substantially about 1 to 1.

One of the advantages of this invention is that pseudovirions can be produced easily for a variety of HPVs using the same reporter and test systems. Another advantage is that the reporter systems can be easily modified, if desired.

An important aspect of this invention is the use of pseudovirions to detect the presence of neutralizing antibodies. Although some information is known about the biology of HPV and other papillomaviruses, they have not yet been successfully cultured *in vitro*. Further, while a large number of animal species are susceptible to papillomavirus infection, each species is only susceptible to its own strains of papillomavirus. Thus, for example, while cottontail rabbit papillomavirus (CRPV) can be successfully studied in the cottontail rabbit and used as a model for the human disease, human papillomavirus cannot be directly studied in the rabbit. Various vaccines based on HPV VLPs are currently under development, but to date neutralization assays have not been available for all strains of HPV.

In the assay of this invention, a pseudovirion of the invention, a sample suspected of containing neutralizing antibodies, and a cell culture are brought into contact. The cells used in this assay may be any cells known to be susceptible to papillomavirus infection. For HPV pseudovirions, the cells may be human carcinoma cells, such as C33A (available from the ATCC), HeLa cells, and the like. If the sample (which in preferred embodiments may be serum from a patient), contains neutralizing antibodies, then the neutralizing antibodies will interact with the pseudovirions and will interfere with the uptake of the pseudovirions the cell. Optionally, a control assay may be performed, wherein the same pseudovirions and cells are used, but the sample is either eliminated, or a sample which is known not to contain neutralizing antibodies is substituted. Another example of a control sample is one from a preimmune source.

In the assay, the decreased uptake of pseudovirions into the cell is correlated to the amount of neutralizing antibody present in the sample. Depending on the reporter gene construct employed, the amount may be quantified. Quantitation of the number of cells expressing the reporter gene, and the extent of expression, can be realized using flow cytometry. Simultaneous measurement of the 520nm/450nm ratio of emission wavelengths is directly related to the proportion of cleaved CCF2 substrate in individual cells, and is independent of substrate concentration or cell dimensions, thus resulting in better signal to noise than is obtainable with either wavelength alone.

In addition, subpopulations of cells that respond to infection could be enriched through fluorescence-activated cell sorting and cultured to expand the population of responsive cells. for example using a quantitative RT PCR assay of a reporter splice variant. These assays of this invention may be miniaturized and/or automated, and used in a 96-well format for high-throughput screens.

The invention also allows labeling of pseudovirions with different reporters to test multivalent neutralizing sera on a mixture of these pseudovirions. This can be used to follow a multivalent vaccine response in a subject.

Another aspect of this invention is the use of pseudovirions to carry different genetic materials into a variety of target cells. Substantially the same linker system is used to construct the pseudovirion. but the reporter gene construct is replaced with a gene construct which expresses a gene of choice.

The following non-limiting Examples are presented to illustrate the invention better.

### EXAMPLE 1

### Expression of HPV 16 VLPs in yeast

HPV 16 VLPs are made essentially as described in WO95/31532.

### Cloning of HPV16 L1 and L2 Genes

Total genomic DNA was extracted from Caski cells (ATCC #CRL 1550) by standard techniques. The DNA was digested with Bst1107I and SphI endonucleases and electrophoresed through a 0.8% low-melting-temperature agarose preparative gel. A region corresponding to DNA of approximately 3.5-kbp was excised from the gel and the agarose was digested with Gelase™ enzyme (Epicentre Technologies, Inc.). The gel-purified DNA was made blunt-ended with T4 DNA polymerase and ligated with blunt-ended, phosphorylated oligodeoxynucleotide linkers that contain a buried HindIII site. The ligation mixture was digested to completion with HindIII and the approximately 3.5-kbp DNA was size-fractionated through an agarose gel as described above. The gel-purified DNA was ligated with pUC18 plasmid DNA that had been digested with HindIII and dephosphorylated. Following transformation of competent *E*. *coli* DH5 cells (BRL), the plasmid library was screened for HPV 16-positive clones by colony hybridization using an antisense ³²P-labeled oligodeoxynucleotide that is complementary to the 3'-end of the HPV-16 L1 gene (5'-GAG AGA TCT TAC AGC TTA CGT TTT TTG CGT TTA GC-3') (SEQ. ID. NO:1). A pUC18 plasmid containing a 3.3-kbp HPV16 genomic fragment was isolated and characterized by restriction enzyme and Southern blot analyses. This plasmid was designated pUC18-HPV16 L1/L2 and contains all of the L1 and L2 coding DNA sequences. Plasmid DNA was prepared using the Qiagen™ Plasmid Maxi kit (Qiagen, Inc.).

### Construction of HPV16 L1 Yeast Expression Vector

The clone, pUC18-HPV16 L1/L2 was used as a template for PCR. The HPV16 L1 gene was amplified by PCR using Vent polymerase (New England Biolabs, Inc.), 10 cycles of amplification (94°C, 1 min; 48°C, 1 min; 72°C, 1 min 45 sec), and the following oligodeoxynucleotide primers which contain flanking BgIII sites (underlined):

The sense primer introduces a yeast non-translated leader sequence immediately upstream to the HPV16 L1 initiating methionine codon (highlighted in bold print). The 1.5-kbp L1 PCR product was digested with BgIII, gel-purified, and ligated with the BamHI digested pC1/1-GAL vector. A pC1/1-GAL plasmid was isolated containing the HPV16 L1 gene and designated, p14049-37-1. The L1 gene in p14049-37-1 was sequenced using the PRISM™ kit (ABI, Inc.) and an ABI Sequencer Model #373A according to the manufacturer's directions. The L1 gene in this isolate was shown to contain 3 nucleotide changes from the corrected, published prototype sequence (Kirnbauer, R. et al. (1993) *J. Virol.* 67: 6929-6936), resulting in two amino acid changes: His-202 to Asp; Thr-266 to Ala. Sequence analysis of the original template DNA confirmed that these changes were also present in the genomic clone pUC18-HPV16 L1/L2 and were not introduced by the PCR.

### Construction of the HPV16 L1 and L2 Yeast Expression Vector

Plasmid p14049-37-1 (pC1/1-GAL+HPV16L1) was digested with SmaI, which cuts between the *GAL10* promoter and the *ADH1* transcription terminator. The 1.4-kbp HPV16 L2 gene was amplified by PCR using the pUC18-HPV16 L1/L2 DNA as template, Vent polymerase (New England Biolabs, Inc.), 10 cycles of PCR amplification (94°C, 1 min; 48°C, 1 min; 72°C, 1 min 45 sec) and the following oligodeoxynucleotide primers which contain flanking SmaI sites (underlined):

The sense primer introduces a yeast non-translated leader sequence immediately upstream to the HPV16 L2 initiating methionine codon (highlighted in bold print). The 1.4-kbp L2 PCR product was digested with SmaI, gel-purified, and ligated with the SmaI digested p14049-37-1 vector. A pC1/1-GAL plasmid containing both the HPV16 L1 and L2 genes was isolated and designated, p14049-42-2. The L2 gene in p14049-42-2 was sequenced using the PRISM™ kit (ABI, Inc.) and an ABI Sequencer Model #373A according to the manufacturer's directions. The L2 gene in this isolate was shown to contain 5 nucleotide changes from the corrected, published prototype sequence (Kimbauer, R. et al. (1993) *supra),* resulting in one amino acid change: Ser-269 to Pro. Sequence analysis of the genomic clone pUC18-HPV16 L1/L2 confirmed that this change was also present in the original template DNA and was not introduced by the PCR.

### Expression of HPV16 L1 and Co-Expression of HPV16 L1 and L2 in Yeast

Plasmids p14049-37-1 (pC1/1-GAL+HPV16 L1) and p14049-42-2 (pC1/1-GAL+HPV16 L1 and L2) were used to transform *S. cerevisiae* strain #1558. The resulting recombinant strains were #1678 (HPV16-L1) and #1679 (HPV16 L1+L2) as shown in the table. Clonal isolates were grown at 30°C in YEHD medium containing 2% galactose for 68-78 hours. After harvesting the cells, the cell pellets were broken with glass beads and cell lysates analyzed for the expression of HPV16 L1 or HPV16 L2 protein by immunoblot analysis. Samples containing 40 mcg of total cellular protein were electrophoresed on 10% Tris-Glycine gels under reducing and denaturing conditions and electroblotted onto nitrocellulose filters. The HPV 16 L1 protein was immunodetected using rabbit polyclonal antisera raised against a trpE-HPV11 L1 fusion protein (D. Brown et al., *Virology* 201:46-54) as primary antibody and HRP-linked, donkey anti-rabbit IgG antibody (Amersham, Inc.) as the secondary antibody. The filters were processed using the chemiluminescern ECL Detection kit (Amersham, Inc.). A 50-55 kDa L1 protein band was detected in all samples except the negative control (pC1/1 without L1 or L2 gene). The L2 protein was detected as a 70 kDa protein band by immunoblot using mouse anti-HPV16 L2 sera raised against trpE-L2 fusion proteins expressed in *E. coli* as primary antibody. Goat anti-mouse IgG HRP-linked (Amersham, Inc.) was used as secondary antibody and the filters were processed as described above.

### EXAMPLE 2

### Purification of VLPs

Purification was done essentially as described in WO 00/09671.

Yeast cells transformed to express VLPs were harvested and frozen for storage at -70°C. Frozen yeast cell suspension was removed from storage and thawed for approximately 3 hours at room temperature followed by approximately 18 hours at 4°C. BENZONASE® (Nycomed Phanna A/S, Copenhagen, Denmark) (2.8 x 105 Units/mL and 0.21 mg protein/mL) was added to the cell suspension to a final concentration of 750 Units per gram of wet cell weight, and in one experiment was reduced to 335 Units per gram wet cell weight. Cells were stirred for 15 minutes, then disrupted by two passes through a sanitized APV Gaulin 30CD homogenizer at chamber pressures of 14,500 to 16.000 psi, resulting in 95% cell disruption. The remaining lysate was gently stirred for 18 hours at 4°C.

Clarification by microfiltration. Cell lysate was clarified by cross-flow microfiltration in a diafiltration mode as follows. Lysate was transferred to a sterile process tank with a 1-inch diameter inlet and outlet ports. The microfilter was a 0.65 micron pore size hollow-fiber filter cartridge of 5 square feet surface area (A/G Technologies #CFP-6-D-8A, Needham, MA) housed in an A/G Technologies FIexStand® Benchtop Pilot Hollow Fiber system. The retentate was diafilteced with 3 volumes of Diafiltration Buffer (below) to produce the clarified lysate. Diafiltration Buffer was 0.2M (Na⁺) MOPS, pH 7.0 + 0.4M NaCl.

Chromatography of clarified lysate. The clarified lysate was fractionated by column chromatography using POROS® 50HS strong cation-exchange chromatography resin (PerSeptive Biosystems, Framingham, MA) packed in a chromatography column. The column was sanitized with 0.5 N NaOH prior to use. The column was equilibrated with HPV Diafiltration Buffer [0.2M (Na⁺)MOPS, pH 7.0 + 0.4M NaCl] at room temperature. The cold (4°C) clarified lysate was pumped onto the column at 125 mL/minute and the column was washed with 8 column volumes of room temperature HPV Column Buffer A [0.05M (Na⁺)MOPS, pH 7.0 + 0.5M NaCl)] at 125 mL/minute with a linear gradient of 100% HPV Column Buffer A to 100% HPV Column Buffer B [0.05M (Na⁺)MOPS, pH 7.0 + 1.5 M NaCl]. Total linear gradient was 10 column volumes and was collected in 10 equal-volume fractions. Following the gradient, the column was washed with two column volumes of room temperature HPV Column Buffer B at 125 mL/minute which were collected in two additional fractions. Fractions were collected in sterile 2-liter plastic bottles and stored at 4°C. Fractions containing the last UV-absorbing peak (A280nm and A230 nm) in the gradient were pooled, filtered using a MILLIPAK-200 disposable filter unit (Millipore, Bedford, MA) and stored at 4°C.

Hydroxyapatite Chromatography All steps were carried out at room temperature. A chromatography column (13 mm ID x 36 mm) packed with Ceramic Hydroxyapatite, Type II (BioRad Cat.#7320081, Hercules, CA), was pre-equilibrated in 50 mM MOPS, pH 7.0 + 1.25 M NaCl. The partially purified HPV solution from the previous step was applied to the column at a linear flow rate of 90 cm/hour. After sample application was complete, the column was washed with eight column volumes of pre-equilibration buffer until the optical density of the column effluent was nearly zero. The HPV product was eluted with a 0% to 100% linear gradient of elution buffer (0.2 M sodium phosphate, pH 7.0 + 1.25M NaCl), also at a linear flow rate of 90 cm/hour. The total volume of the gradient was four column volumes. Fractions containing the vaccine product were identified by RIA and Bradford protein assay. The protein concentration of the product was 100 µg/mL.

Assays : Bradford protein assays were performed using Coomassie Plus Assay Reagent (Pierce, Rockford, IL) using bovine serum albumin (BSA) as a standard. Lowry protein assays were performed according to the procedure of Lowry et al 1951 *J*. *Biol*. *Chem*. 193:265-270 using BSA as a calibration standard. Antigen was assayed by a multilayered ELISA using a monoclonal antibody that recognized a conformational epitope of the VLP. Microtiter plates were coated with polyclonal goat anti-HPV VLP antibodies. Standard and test samples were diluted with PBS containing 1% w/v BSA, 0.1% TWEEN-20, and 0.1% sodium azide and were added to the wells where antigen was captured by the plate-bound antibodies. Monoclonal anti-HPV L1 VLP antibody (Chemicon, Temecula, CA) was added to the wells to bind the antigen captured by the plate-bound antibodies. The monoclonal anti-HPV VLP antibodies were detected by horseradish peroxidase-conjugated anti-mouse IgG antibodies. A chromogenic substrate for horseradish peroxidase, 3,3',5,5'-tetramethylbenzidine (Pierce) was added and absorbance at 450 nm was proportional to the concentration of L1 VLP in the sample.

### EXAMPLE 3

### Generation of the beta-lactamase reporter and coupling to HPV16 VLPs to produce "pseudovirions"

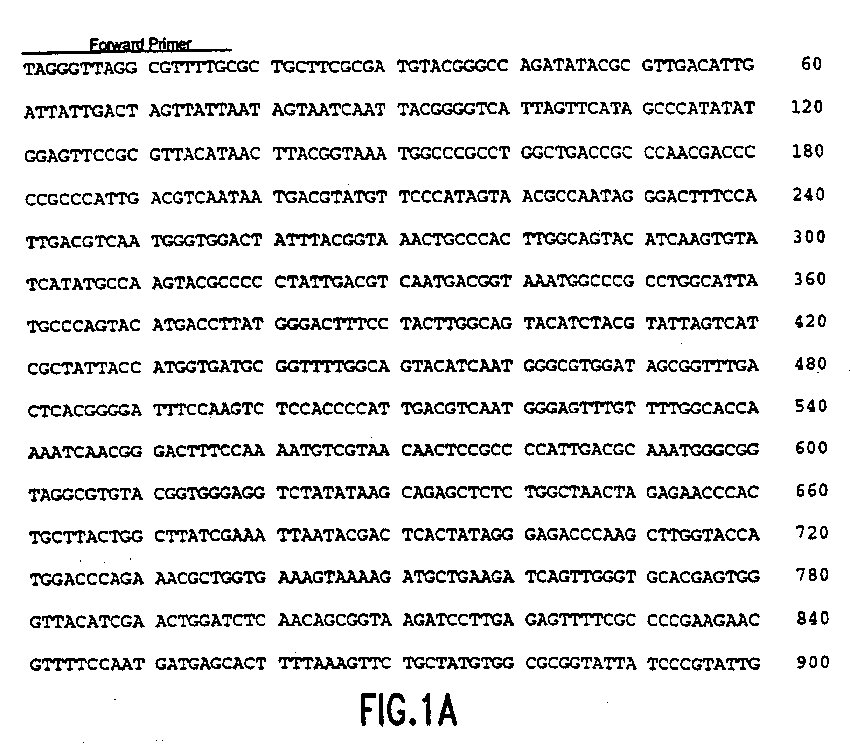

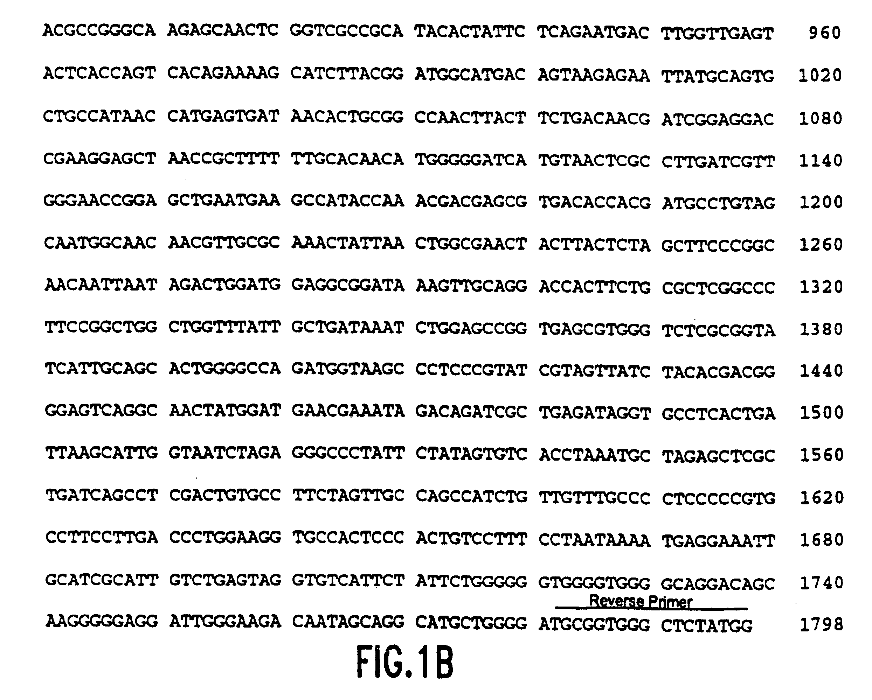

The beta-lactamase reporter gene was constructed as follows: 5'-amine modified oligos were designed, synthesized (Midland Certified Reagents; Midland, TX) and used to amplify the section of the Aurora vector pcDNA3-BLAM (Zlokarnik et al 1998 *Science* 279:84-88) containing the CMV promoter + BLAM + poly-A tail (Figure 1). Primers for both coupling orientations can be used (i.e., amine at 5' end of sense strand or amine at 5' end of antisense strand). The downstream primers were 5'-biotinylated in order to reduce degradation by DNA exonuclease in the final assay, and to provide the ability to detect DNA. For primer sequences see Table 1.

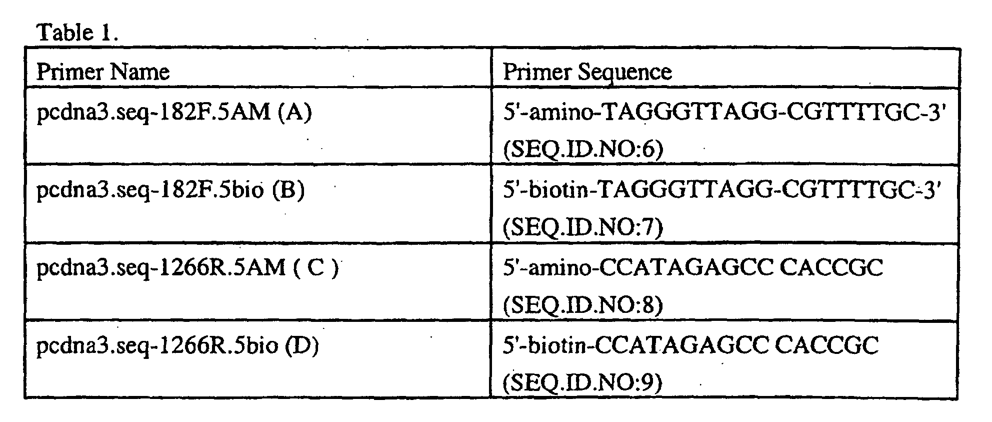

Primers A and B were initially used to amplify a 1798 base pair (bp) sequence encoding the 591 bp human CMV promoter, the 809 bp beta-lactamase and the 135 bp bovine growth hormone poly A terminator sequences. PCR products were purified using gel filtration (spin-columns with a silica-gel membrane, QIAGEN,Inc.), quantitated by UV spectrometry at OD 260 nm, and conjugated to the NHS-ester of the heterobifunctional crosslinker SMCC (succinimidyl-maleimidomethyl-cyclohexane-carboxylate) (Pierce) according to the manufacturer's instructions for 1 hr at 20°C.

The conjugated reporter gene was purified by gel filtration (Microspin G-25, Pharmacia), then reacted to free sulfhydryls on concentrated HPV16 VLPs ( 0.97 mg/mL ), via the maleimide group (approx. 1 free sulfhydryl per L1 monomer).

The pseudovirions were then sterile filtered to be used in the neutralization assay. Initial experiments indicated that this low coupling ratio of reporter to VLP was beneficial (data not shown). The reaction took place at 4°C for 12 hours.

### EXAMPLE 4

### Infection of C33A cells with HPV pseudovirions

2000 C33 A cells (ATCC #HTB 31) were cultured for 3 days at 37°C in complete DMEM (Dulbecco's Modified Eagle Medium (GIBCO BRL) containing Penicillin and Streptomycin (100 U/mL and 100 mcg/mL, respectively) and 10% fetal calf serum. Before infection, the serum containing medium was removed and 200 ng HPV 16 pseudovirions in 50 mcL of opti-MEM (GIBCO BRL) were added. 24 hours after infection, the cells were fed with 100 mcL of complete DMEM medium. Two days after infection, beta-lactamase reporter gene expression was detected in infected CA33A cells using CCF2 (Aurora), a substrate for the beta-lactamase. (Zlokarnik, *supra).*

Briefly, cells were washed with opti-MEM medium before 50 mcL of loading buffer containing CCF2 was added to each well of a 96 well plate (FALCON, Becton-Dickinson). CCF2 final concentration was 5 microM. Plates were incubated for 3h at room temperature. Conversion of CCF2 in cells expressing beta-lactamase was followed by monitoring the shift in fluorescence emission wavelength to blue at 460 nm using a fluorescent microscope (Olympus, Mod. IX70). On average, 204 +/- 55 (n=12) blue cells could be detected two days after infection.

### EXAMPLE 5

### Neutralization of HPV16 pseudovirion infection by monoclonal antibodies and immune sera from HPV16 VLP immunized individuals

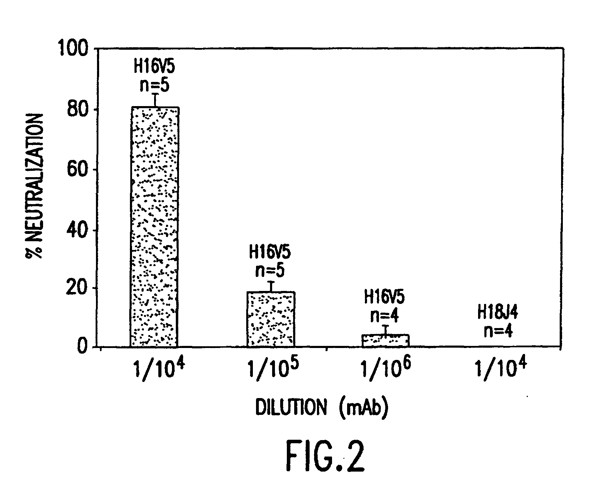

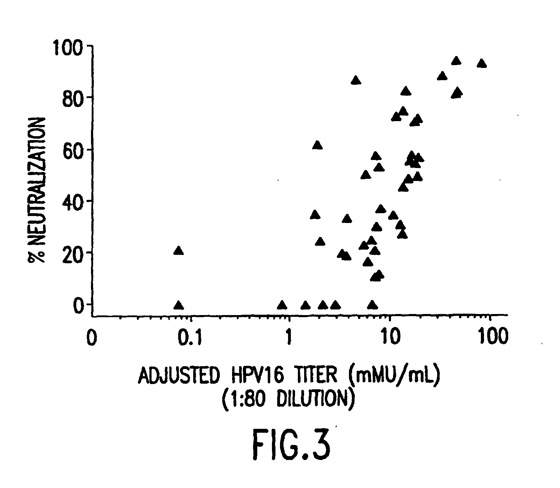

2000 C33A cells/well (ATCC #HTB 31) were cultured in 96 well plates (FALCON, Becton-Dickinson) for 3 days at 37°C in complete DMEM (GIBCO BRL). 25 mcl of 1:40 dilutions of either (i) human pre-immune sera, (ii) immune sera from individuals immunized with HPV16 VLPs, (iii) immune sera from individuals immunized with other HPV VLPs, or (iv) various dilutions of HPV VLP-specific monoclonal antibodies were added to the wells, followed by 25 mcl of medium containing 200 ng of pseudovirions. The plates were incubated at 37°C in a CO₂ incubator (5% CO₂) and then analyzed as described in Example 3. Percent neutralization was determined by calculating the percentage of reduction of blue cells in wells containing HPV16 neutralizing monoclonal antibodies or human immune sera versus wells containing non-specific monoclonal antibodies or pre-immune sera. Figure 2 shows the percent neutralization of HPV16 pseudovirions by serial dilutions of ascites containing the known HPV16 neutralizing monoclonal antibody H16. V5. A 1:10,000 dilution of ascites containing the nonspecific monoclonal antibody H18.J4 was not neutralizing. Using the described assay, a panel of 52 sera of individuals immunized with HPV16 VLPs was tested at a 1:80 final serum dilution. Most of the immune sera were neutralizing in this assay (Figure 3). In addition, a good correlation was seen between HPV16 VLP-specific antibody titers determined by an HPV16 RIA antibody test and % neutralization (Figure 3).

### EXAMPLE 6

Polystyrene beads (1/4 inch with specular finish) were pre-coated with normal goat serum at a 1:1000 dilution in phosphate-buffered saline (PBS). Batches of 3000 beads, 7.5 beads/ml, were incubated overnight at 4°C. Beads were then washed in 5 volumes of deionized water, blotted dry on paper towels and permitted to air dry on a stainless steel tray. HPV 16 L1 VLP were coated onto the pre-coated beads using MOPS buffer (50 mM MOPS + 0.5 M NaCl, pH 7.0) containing 50 ng of VLP per ml (5 beads per ml). Antigen and buffer were mixed first in a Corning® media bottle then the beads were added. The bottle containing the coating antigen and beads was secured onto a Cole Palmer rotator, coating was for 1 hour at room temperature at 5 rpm. Beads were washed in 5 volumes of MOPS buffer and sufficient buffer was added to keep beads submerged. They were then stored until needed at 4°C for a maximum of 4 months.

The HPV16 L1 VLP antibody RIA is a competitive antibody assay, where antibodies compete for limited number of epitopes with a limited amount of the conformational specific monoclonal antibody H16.V5. Ascites containing H16.V5 were used at a 1:800,000 dilution in PBS + 1% bovine serum albumin + 0.05% Tween 20. Equal volumes (100mclL) of sample serum and diluted H16.V5 were mixed in a well of a 20 well Abbott assay plate. Then a single HPV16 antigen-coated bead was added to the assay well. Samples were assayed in duplicates.

Assay plates were sealed with adhesive sheets and left to incubate at room temperature 17 to 24 hours. Plates were washed with deionized water using an Abbott plate washer. The amount of mouse MAB that successfully bound to the HPV16 VLP-coated bead was detected with ¹²⁵I [Goat anti-mouse IgG] (NEN Life Sciences, Boston, MA). Plates were sealed with fresh adhesive sheet and incubated at 37°C for 2 to 2.5 hours. Plates were again washed with deionized water. The beads were then transferred to carrier tubes and read on a gamma counter. Relative inhibition of the H16.V5 binding was compared to a standard curve using a four parameter logistic curve fit. The standard curve was derived using an HPV16 immune serum with an arbitrarily assigned number of units (mMU/mL).

## Claims

1. A human papillomavirus (HPV) pseudovirion comprising a virus-like particle (VLP) covalently linked to a reporter gene construct.

2. A pseudovirion according to Claim 1 wherein the HPV is selected from the group consisting of: HPV6a, HPV6b, HPV11, HPV16, HPV18, HPV31, HPV33, HPV35, HPV42, HPV43, HPV44, HPV45, HPV51, HPV52 and HPV56.

3. A pseudovirion according to Claim 1 wherein the VLP is comprised of L1 or L1+L2 protein.

4. A pseudovirion according to Claim 3 wherein the reporter gene construct is selected from the group consisting of: beta-lactamase gene constructs, beta galactosidase gene constructs, luciferase gene constructs and green fluorescent protein gene constructs.

5. A pseudovirion comprising a HPV VLP, a heterobifunctional crosslinker and a reporter gene construct.

6. A pseudovirion according to Claim 5 wherein the molar ratio of the reporter gene construct to VLP is from about 0. 1 to 50.

7. A pseudovirion according to Claim 5, wherein the molar ratio of the reporter gene construct to VLP is about 1 to 5.

8. A pseudovirion according to Claim 5 wherein the molar ratio of the reporter gene construct to VLP is about 1 to 1.

9. An assay to determine if anti-HPV neutralizing antibodies are present in a sample, comprising the steps of:
a) contacting human papillomavirus pseudovirions, a cell culture susceptible of being infected by the pseudovirions, and the sample, wherein the pseudovirions comprise a virus-like particle (VLP) covalently linked to a reporter gene construct; and
b) determining if cell uptake of pseudovirions is inhibited, wherein inhibition of uptake is indicative of anti-HPV neutralizing antibodies in the sample.

10. An assay according to Claim 9 wherein the sample comprises serum from a human.

11. An assay according to Claim 10 further comprising the step of comparing the results to those obtained using a control assay.

12. An assay according to Claim 9 wherein the pseudovirions comprise a beta-lactamase gene construct.

## Patentansprüche

1. Human-Papillomavirus (HPV)-Pseudovirion, umfassend ein virusähnliches Partikel (VLP) in kovalenter Verknüpfung mit einem Reportergen-Konstrukt.

2. Pseudovirion nach Anspruch 1, wobei das HPV aus der Gruppe, bestehend aus: HPV6a, HPV6b, HPV11, HPV16, HPV18, HPV31, HPV33, HPV35, HPV42, HPV43, HPV44, HPV45, HPV51, HPV52 und HPV56 ausgewählt ist.

3. Pseudovirion nach Anspruch 1, wobei das VLP L1- oder L1+L2-Protein umfasst.

4. Pseudovirion nach Anspruch 3, wobei das Reportergen-Konstrukt aus der Gruppe, die aus Beta-Lactamasegen-Konstrukten, Beta-Galactosidasegen-Konstrukten, Luciferasegen-Konstrukten und Konstrukten mit dem Gen des Grünen Fluoreszenzproteins besteht, ausgewählt ist.

5. Pseudovirion, umfassend ein HPV-VLP, ein heterobifunktionelles Verknüpfungsmittel (Linker) und ein Reportergen-Konstrukt.

6. Pseudovirion nach Anspruch 5, wobei das Molverhältnis des Reportergen-Konstrukts zu VLP etwa 0,1 bis 50 beträgt.

7. Pseudovirion nach Anspruch 5, wobei das Molverhältnis des Reportergen-Konstrukts zu VLP etwa 1 bis 5 beträgt.

8. Pseudovirion nach Anspruch 5, wobei das Molverhältnis des Reportergen-Konstrukts zu VLP etwa 1 zu 1 beträgt.

9. Assay zur Feststellung, ob neutralisierende Anti-HPV-Antikörper in einer Probe vorhanden sind, umfassend die Schritte:
a) Kontaktieren von Human-Papillomavirus-Pseudovirionen, einer Zellkultur, die einer Infektion durch die Pseudovirionen zugänglich ist, und der Probe, wobei die Pseudovirionen ein virusähnliches Partikel (VLP) in kovalenter Verknüpfung mit einem Reportergen-Konstrukt umfassen; und
b) Feststellen, ob die Zellaufnahme von Pseudovirionen inhibiert ist, wobei eine Inhibierung der Aufnahme neutralisierende Anti-HPV-Antikörper in der Probe anzeigt.

10. Assay nach Anspruch 9, wobei die Probe Serum von einem Menschen umfasst.

11. Assay nach Anspruch 10, ferner umfassend den Schritt des Vergleichs der Ergebnisse mit den bei Einsatz eines Kontroll-Assays erhaltenen.

12. Assay nach Anspruch 9, wobei die Pseudovirionen ein Beta-Lactamasegen-Konstrukt umfassen.

## Revendications

1. Pseudovirion du papillomavirus humain (HPV) comprenant une particule de type viral (PTV) liée à un gène reporter chimère d'une manière covalente.

2. Pseudovirion selon la revendication 1 dans lequel le HPV est choisi dans le groupe comprenant : HPV6a, HPV6b, HPV11, HPV16, HPV18, HPV31, HPV33, HPV35, HPV42, HPV43, HPV44, HPV45, HPV51, HPV52 et HPV56.

3. Pseudovirion selon la revendication 1 dans lequel la PTV est constituée de la protéine L1 ou de la protéine L1+L2.

4. Pseudovirion selon la revendication 3 dans lequel le gène reporter chimère est choisi dans le groupe comprenant : les gènes chimères de la bêta-lactamase, les gènes chimères de la bêta-galactosidase, les gènes chimères de la luciférase et les gènes chimères de la protéine verte fluorescente.

5. Pseudovirion comprenant une PTV du HPV, un agent réticulant hétérobifonctionnel et un gène reporter chimérique.

6. Pseudovirion selon la revendication 5 dans lequel le ratio molaire du gène reporter chimérique sur la PTV est d'environ 0,1 à 50.

7. Pseudovirion selon la revendication 5 dans lequel le ratio molaire du gène reporter chimérique sur la PTV est d'environ 1 à 5.

8. Pseudovirion selon la revendication 5 dans lequel le ratio molaire du gène reporter chimérique sur la PTV est d'environ 1 à 1.

9. Dosage visant à déterminer si des anticorps neutralisants anti-HPV sont présents dans un échantillon, comprenant les étapes consistant à :
a) mettre en contact des pseudovirions du papillomavirus humain, une culture cellulaire susceptible d'être infectée par les pseudovirions et l'échantillon, sachant que les pseudovirions comprennent une particule de type viral (PTV) liée à un gène reporter chimère d'une manière covalente ; et
b) déterminer si la capture cellulaire des pseudovirions est inhibée, l'inhibition de la capture étant indicatrice de la présence d'anticorps neutralisants anti-HPV dans l'échantillon.

10. Dosage selon la revendication 9 dans lequel l'échantillon comprend du sérum provenant d'un être humain.

11. Dosage selon la revendication 10 comprenant en outre l'étape consistant à comparer les résultats à ceux obtenus avec un dosage de contrôle.

12. Dosage selon la revendication 9 dans lequel les pseudovirions comprennent un gène chimérique de la bêta-lactamase.


### SEQUENCE LISTING

<110> Merck & Co., Inc.
<120> NEUTRALIZING ASSAY USING HUMAN PAPILLOMAVIRUS VIRUS-LIKE PARTICLES
<130> 20342 PCT
<140> PCT/US99/30297
   <141> 1999-12-20
<150> 60/113,388
   <151> 1998-12-23
<160> 10
<170> FastSEQ for Windows Version 4.0
<210> 1
   <211> 35
   <212> DNA
   <213> Artificial Sequence
   <220>
   <223> Probe
<400> 1
   gagagatctt acagcttacg ttttttgcgt ttagc 35
<210> 2
   <211> 47
   <212> DNA
   <213> Artificial Sequence
   <220>
   <223> PCR primer
<400> 2
   ctcagatctc acaaaacaaa atgtctcttt ggctgcctat gtaggcc 47
<210> 3
   <211> 35
   <212> DNA
   <213> Artificial Sequence
   <220>
   <223> PCR primer
<400> 3
   gagagatctt acagcttacg ttttttgcgt ttagc 35
<210> 4
   <211> 45
   <212> DNA
   <213> Artificial Sequence
   <220>
   <223> PCR primer
<400> 4
   tcccccgggc acaaaacaaa atgcgacaca aacgttctgc aaaac 45
<210> 5
   <211> 33
   <212> DNA
   <213> Artificial Sequence
   <220>
   <223> PCR primer
<400> 5
   tcccccgggc taggcagcca aagagacatc tga 33
<210> 6
   <211> 18
   <212> DNA
   <213> Artificial Sequence
   <220>
   <223> PCR primer
<400> 6
   tagggttagg cgttttgc 18
<210> 7
   <211> 18
   <212> DNA
   <213> Artificial Sequence
   <220>
   <223> PCR primer
<400> 7
   tagggttagg cgttttgc 18
<210> 8
   <211> 16
   <212> DNA
   <213> Artificial Sequence
   <220>
   <223> PCR primer
<400> 8
   ccatagagcc caccgc 16
<210> 9
   <211> 16
   <212> DNA
   <213> Artificial Sequence
   <220>
   <223> PCR primer
<400> 9
   ccatagagcc caccgc 16
<210> 10
   <211> 1798
   <212> DNA
   <213> Artificial Sequence
   <220>
   <223> Reporter gene based on Beta-lactamase gene
<400> 10
   tagggttagg cgttttgcgc tgcttcgcga tgtacgggcc agatatacgc gttgacattg      60
   attattgact agttattaat agtaatcaat tacggggtca ttagttcata gcccatatat     120
   ggagttccgc gttacataac ttacggtaaa tggcccgcct ggctgaccgc ccaacgaccc     180
   ccgcccattg acgtcaataa tgacgtatgt tcccatagta acgccaatag ggactttcca     240
   ttgacgtcaa tgggtggact atttacggta aactgcccac ttggcagtac atcaagtgta     300
   tcatatgcca agtacgcccc ctattgacgt caatgacggt aaatggcccg cctggcatta     360
   tgcccagtac atgaccttat gggactttcc tacttggcag tacatctacg tattagtcat     420
   cgctattacc atggtgatgc ggttttggca gtacatcaat gggcgtggat agcggtttga     480
   ctcacgggga tttccaagtc tccaccccat tgacgtcaat gggagtttgt tttggcacca     540
   aaatcaacgg gactttccaa aatgtcgtaa caactccgcc ccattgacgc aaatgggcgg     600
   taggcgtgta cggtgggagg tctatataag cagagctctc tggctaacta gagaacccac     660
   tgcttactgg cttatcgaaa ttaatacgac tcactatagg gagacccaag cttggtacca     720
   tggacccaga aacgctggtg aaagtaaaag atgctgaaga tcagttgggt gcacgagtgg     780
   gttacatcga actggatctc aacagcggta agatccttga gagttttcgc cccgaagaac     840
   gttttccaat gatgagcact tttaaagttc tgctatgtgg cgcggtatta tcccgtattg     900
   acgccgggca agagcaactc ggtcgccgca tacactattc tcagaatgac ttggttgagt     960
   actcaccagt cacagaaaag catcttacgg atggcatgac agtaagagaa ttatgcagtg    1020
   ctgccataac catgagtgat aacactgcgg ccaacttact tctgacaacg atcggaggac    1080
   cgaaggagct aaccgctttt ttgcacaaca tgggggatca tgtaactcgc cttgatcgtt    1140
   gggaaccgga gctgaatgaa gccataccaa acgacgagcg tgacaccacg atgcctgtag    1200
   caatggcaac aacgttgcgc aaactattaa ctggcgaact acttactcta gcttcccggc    1260
   aacaattaat agactggatg gaggcggata aagttgcagg accacttctg cgctcggccc    1320
   ttccggctgg ctggtttatt gctgataaat ctggagccgg tgagcgtggg tctcgcggta    1380
   tcattgcagc actggggcca gatggtaagc cctcccgtat cgtagttatc tacacgacgg    1440
   ggagtcaggc aactatggat gaacgaaata gacagatcgc tgagataggt gcctcactga    1500
   ttaagcattg gtaatctaga gggccctatt ctatagtgtc acctaaatgc tagagctcgc    1560
   tgatcagcct cgactgtgcc ttctagttgc cagccatctg ttgtttgccc ctcccccgtg    1620
   ccttccttga ccctggaagg tgccactccc actgtccttt cctaataaaa tgaggaaatt    1680
   gcatcgcatt gtctgagtag gtgtcattct attctggggg gtggggtggg gcaggacagc    1740
   aagggggagg attgggaaga caatagcagg catgctgggg atgcggtggg ctctatgg      1798